# EUROPÄISCHE PATENTSCHRIFT

(11) **EP 0 949 256 B1**
(45) Veröffentlichungstag und Bekanntmachung des Hinweises auf die Patenterteilung: **28.08.2002**
(21) Anmeldenummer: 99105411.5
(22) Anmeldetag: 17.03.1999
(51) Int. Cl.: C07D 311/76

(54) **Verfahren zur Herstellung von Isochroman-3-onen**
Process for the preparation of isochroman-3-ones
Procédé de préparation d'isochromane-3-ones

(30) Priorität: 06.04.1998 DE 19815323
(43) Veröffentlichungstag der Anmeldung: 13.10.1999
(73) Patentinhaber: Clariant GmbH, 65929 Frankfurt am Main (DE)
(72) Erfinder: Geissler, Holger Dr., 55116 Mainz (DE); Pfirmann, Ralf Dr., 64347 Griesheim (DE)

(56) Entgegenhaltungen:
- EP-A- 0 834 497
- WO-A-98/56784
- WO-A-99/10335
- R. SUSTMANN UND H.-G. KORTH: 'Methoden der Organischen Chemie (Houben-Weyl), Band E5', 30 Mai 1985, GEORG THIEME VERLAG, STUTTGART * Seite 302 - Seite 303 *
- H.G.O. BECKER ET AL.: 'Organikum', 1990, DEUTSCHER VERLAG DER WISSENSCHAFTEN, BERLIN * Seite 180 *

## Beschreibung

Die vorliegende Erfindung betrifft ein vorteilhaftes Verfahren zur Herstellung von Isochroman-3-onen.

lsochroman-3-on ist bei der Synthese von Pharmazeutika und Pflanzenschutzmitteln als Zwischenprodukt von großem Interesse.

Aus der WO 97/12864 geht beispielsweise die Verwendung von Isochroman-3-on als Zwischenprodukt bei der Herstellung von Fungiziden und Pestiziden hervor.

Die Qualität von traditionellen chemischen Prozessen ist in der Regel durch die Raum/Zeitausbeute gegeben. In katalytisch chemischen Prozessen wird dagegen in der Regel die katalytische Wechselzahl (sogenannte "turnover number" oder "TON", d.h. der Wert, der angibt, wie oft ein Katalysatorteilchen in der Reaktion benutzt wird) und die katalytische Wechselfrequenz (sogenannte "turnover frequence" oder "TOF", d.h. der Wert, der angibt, wie oft ein Katalysatorteilchen in einer Stunde in der Reaktion benutzt wird) als Qualitätsmerkmal herangezogen. Die TON und TOF geben im Vergleich mit der Raum/Zeitausbeute zusätzlich Information über die Qualität des in der Reaktion eingesetzten Katalysators.

In der Literatur sind verschiedene Verfahren zur Herstellung von Isochroman-3-on bekannt.

So beschreibt Yamamoto in Tetrahedron Lett. 1997, Vol. 38, 3747 bis 3750 eine Synthese von lsochroman-3-on durch Umsetzung von 1,2-Bis-hydroxymethyl-benzol und Kohlenmonoxid in Gegenwart von 1 mol-% eines Palladiumkatalysators und 10 mol-% Jodwasserstoff. Man erhält bei 90°C und 9 MPa Kohlenmonoxid-Druck in Aceton-Wasser als Lösungsmittel nach 42 Stunden Reaktionszeit Isochroman-3-on in isolierter Form mit 56 % Ausbeute.

Als Nachteile dieses Verfahrens sind die Anwesenheit des sehr korrosiv wirkenden Jodwasserstoffs und die recht lange Reaktionszeit zu erwähnen.

Stille beschreibt in J. Am. Chem. Soc. 1980, Vol. 102, 4193 bis 4198 die Synthese von lsochroman-3-on durch Umsetzung von ortho-Brommethylbenzylalkohol, Kohlenmonoxid und Kaliumcarbonat in Gegenwart von 1,6 mol-% eines Palladiumkatalysators und einem Tropfen Hydrazin in Tetrahydrofuran als Lösungsmittel. Man erhält nach 24 Stunden bei 25°C und 0,1 MPa Kohlenmonoxid-Druck lsochroman-3-on in isolierter Form mit einer Ausbeute von 71 %.

Von Nachteil ist, daß der als Ausgangsstoff benötigte ortho-Brommethylbenzylalkohol nicht einfach zugänglich ist. Zudem erschwert die Verwendung von Kaliumcarbonat eine einfache Verfahrensdurchführung (Freisetzen von CO₂). Ferner ist eine vergleichsweise lange Reaktionszeit in Kauf zu nehmen.

Aus der WO 97/00850 A1 geht ein zweistufiges Verfahren zur Herstellung von lsochroman-3-on-Derivaten hervor, wobei zunächst ein 1,2-Bis-halomethylbenzolderivat der allgemeinen Formel (A) 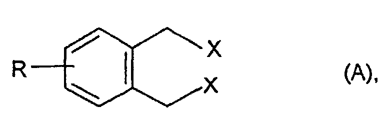 worin R für H, ein Halogen, einen C₁-C₆-Alkyl- oder C₁ -C₆-Alkoxy-Rest und X für ein Halogen steht, Kohlenmonoxid und Wasser in Gegenwart eines Wasserstoffhalogenidaufnahmemittels und eines Katalysators in einem organischen Lösungsmittel umgesetzt werden und das als Zwischenprodukt auftretende Salz der ortho-Hydroxymethylphenylessigsäure der allgemeinen Formel (B), worin M für ein Alkali- oder Erdalkalimetall und n für 1 oder 2 steht, 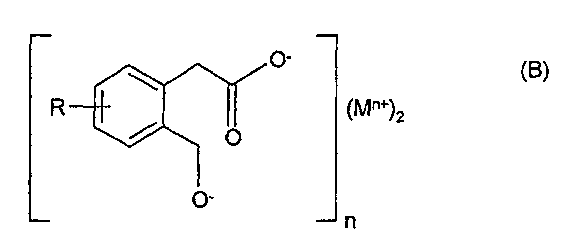 anschließend mit einer Säure behandelt und in das entsprechende Isochroman-3-on umgewandelt wird. Als Katalysator kommen Palladium-, Kobalt- und Eisenkatalysatoren in Betracht. Als Wasserstoffhalogenidaufnahmemittel kommen Basen, insbesondere anorganische Basen, beispielsweise Calciumhydroxid zum Einsatz. In der zweiten Reaktionsstufe dieses Verfahrens wird als Säure beispielsweise Salzsäure verwendet, um die Umwandlung des Salzes des ortho-Hydroxymethylphenylessigsäurederivates der Formel (B) in das entsprechende lsochroman-3-on herbeizuführen. Die maximale TOF beträgt 153 x h⁻¹; TON = 153; Ausbeute 76,7 % (vgl. Ausführungsbeispiel 4). Die maximale TON beträgt 170 (TOF = 24 x h⁻¹); Ausbeute 84,7 % (vgl. Ausführungsbeispiel 17).

Nach diesem Verfahren kann zwar eine Ausbeute von bis zu 87,4 % an Isochroman-3-on erzielt werden, wobei jedoch eine vergleichsweise geringe Menge von 8,75 g α,α'-ortho-Xylylendichlorid (1,2-Bis-chlormethylbenzol) in nicht weniger als 100 g tert.-Butanol umgesetzt wird. Zur weiteren Aufarbeitung wird das Reaktionsgemisch mit Wasser versetzt, unlösliche Feststoffe werden durch Filtration abgetrennt und es wird mehrfach mit Ether extrahiert. Nach Ansäuem mit konzentrierter Salzsäure wird erneut mit Ether extrahiert und aus den gesammelten Etherfraktionen Isochroman-3-on gewonnen (TON = 87; TOF = 4,2 x h⁻¹; vgl auch Ausführungsbeispiel 5).

Bedingt durch Einsatz von Basen im ersten Schritt des Verfahrens und durch das Ansäuern im zweiten Schritt entstehen nicht weniger als 3 Äquivalente einwertiges Salz pro Äquivalent lsochroman-3-on. Nachteilig in diesem Verfahren ist einerseits der Einsatz großer Mengen Lösungsmittel und die Bildung hoher Salzmengen und andererseits die Zweistufigkeit des Verfahrens und die zahlreichen Reinigungs- und Extraktionsschritte sowie die wiederholte Verwendung von Ether als Extraktionsmittel.

Im Hinblick auf die Nachteile der vorstehend geschilderten Verfahren liegt der vorliegenden Erfindung die Aufgabe zugrunde, ein neues Verfahren zur Herstellung von lsochroman-3-onen bereitzustellen, das sich zum einen mit vergleichsweise geringem Aufwand ausführen läßt, zum anderen die zuvor beschriebenen Nachteile der Verfahren des Standes der Technik vermeidet und das gewünschte Produkt in guter Ausbeute und hoher Reinheit zugänglich macht.

Diese Aufgabe wird gelöst durch ein Verfahren zur Herstellung eines Isochroman-3-ons der Formel (I) 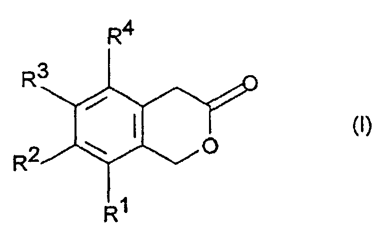 durch Umsetzung eines 1,2-Bis-halogenmethylbenzols der Formel (II) 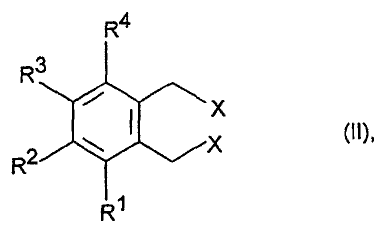 worin X Chlor, Brom oder Jod ist, mit Kohlenmonoxid und Wasser bei einem CO-Druck von 0,1 bis 50 MPa und einer Temperatur von 20 bis 200°C in Anwesenheit oder Abwesenheit eines ionischen Halogenids, in Gegenwart eines Palladiumkatalysators und eines dipolar aprotischen Lösungsmittels, wobei in den Formeln (I) und (II) die Reste R¹, R², R³ und R⁴ unabhängig voneinander darstellen:
ein Wasserstoff- oder Fluoratom;
eine HO₂CCH=CH-, NC- oder F₃C-Gruppe;
einen Alkyl-, Alkoxy- oder Acyloxyrest, mit jeweils 1 bis 18 Kohlenstoffatomen; oder
einen C₆-C₁₈-Aryloxy-, Aryl- oder Heteroarylrest, wobei als Heteroatome 1 bis 3 Atome aus der Gruppe O, N und/oder S vorhanden sind;
einen R⁵₂P(=O)-, R⁶C(=O)-, R⁶OC(=O)-, R⁶OC(=O)CH=CH-, R⁷C(=O)-, R⁷OC(=O)CH=CH- oder R⁷₂P(=O)-Rest; worin R⁵ einen C₁-C₄-Alkylrest, R⁶ einen C₁-C₁₈-Alkylrest und R⁷ einen C₆-C₁₈-Arylrest darstellen; oder worin mindestens einer der Reste R¹, R², R³ und R⁴ durch einen Rest R⁸ substituiert ist, wobei R⁸ die gleiche Bedeutung hat wie R¹; oder worin mindestens zwei der Reste R¹, R², R³ und R⁴ untereinander verknüpft sind und mindestens einen aliphatischen oder aromatischen Ring mit 5 bis 18 C-Atomen bilden.

Das erfindungsgemäße Verfahren ermöglicht es, das 1,2-Bis-halogenmethylbenzol der Formel (II) in Konzentrationen, die signifikant höher als im Verfahren gemäß der WO 97/00850 A1 liegen, umzusetzen. Dadurch wird vorteilhafterweise die Raum/Zeitausbeute erhöht und eine technische Durchführung in entsprechendem Maße begünstigt.
Ein weiterer Vorteil besteht darin, daß im Vergleich zum Verfahren der WO 97/00850 A1 nicht das Salz der Formel (B) zu bilden ist und auf den zweiten Reaktionsschritt unter Zusatz von Säure verzichtet werden kann.

Die Reste R¹, R², R³ und R⁴ bedeuten insbesondere unabhängig voneinander Wasserstoff, Fluor, C₁-C₄-Alkyl oder C₁-C₄-Alkoxy oder zwei der Reste R¹, R², R³ und R⁴ sind untereinander verknüpft und bilden einen aliphatischen oder aromatischen Ring mit 5 bis 10 C-Atomen. Vorzugsweise stehen R¹, R², R³ und R⁴ unabhängig voneinander für Wasserstoff, Fluor oder C₁-C₄-Alkyl.

In den Formeln (I) und (II) bedeuten zwei, drei oder vier, insbesondere drei oder vier der Reste R¹, R², R³ und R⁴ Wasserstoff.

Man kann in das Verfahren mit gutem Erfolg ein 1,2-Bis-halogenmethylbenzol der Formel (II), worin X für Chlor oder Brom, insbesondere für Chlor steht, einsetzen.

Wie eingangs erwähnt, kann man das Verfahren in Anwesenheit oder Abwesenheit eines ionischen Halogenids durchführen.

Üblicherweise ist das ionische Halogenid ein Alkali-, Ammonium- oder Phosphoniumhalogenid, insbesondere ein Alkali- oder Ammoniumhalogenid, wobei Halogenid die Bedeutung Chlorid, Bromid oder Jodid, insbesondere Chlorid oder Bromid, bevorzugt Chlorid hat.

Man kann als ionisches Halogenid Ammoniumbromid, Lithiumbromid, Natriumbromid, Kaliumbromid, Tetrabutylphosphoniumbromid, Ammoniumchlorid, Dimethylammoniumchlorid, Diethanolammoniumchlorid, Lithiumchlorid, Natriumchlorid, Kaliumchlorid, Tetrabutylphosphoniumchlorid, Ammoniumjodid, Lithiumjodid, Natriumjodid, Kaliumjodid und/oder Tetrabutylphosphoniumjodid, insbesondere Lithiumchlorid, Ammoniumchlorid, Dimethylammoniumchlorid, und/oder Diethanolammoniumchlorid einsetzen.

An dieser Stelle sei darauf hingewiesen, daß man auf die Anwesenheit des ionischen Halogenids verzichten und das Verfahren insbesondere in Abwesenheit des ionischen Halogenids durchführen kann.

Man kann in das Verfahren einen Palladiumkatalysator, der auf einem Trägermaterial aufgebrachtes Palladium enthält, einsetzen. Ein derartiger Palladium-Träger-Katalysator hat den Vorzug, daß er sich aus dem Reaktionsgemisch, beispielsweise durch Filtration, auf einfache Weise abtrennen läßt.

In einer Reihe von Fällen hat es sich bewährt, daß der Palladiumkatalysator mindestens eine Palladium-(II)-Verbindung, insbesondere PdCl₂, PdBr₂ oder Pd(OAc)₂ bevorzugt PdCl₂, oder mindestens eine Palladium-(0)-Verbindung, insbesondere Pd₂dba₃, worin dba für Dibenzylidenaceton steht, Pd(P(C₆H₅)₃)₄ oder Pd(η⁴-C₈H₁₂)₂, bevorzugt Pd₂dba₃ enthält.

In einer Reihe von Fällen hat es sich ferner als günstig erwiesen, daß der Palladiumkatalysator zusätzlich einen Liganden, insbesondere eine Phosphinverbindung, enthält.

Als Phosphinverbindung kommt beispielsweise ein Monophosphin, insbesondere ein Tri-(C₁-C₆-alkyl)phosphin oder ein Triarylphosphin, oder ein Diphosphin in Betracht. Mit gutem Erfolg kann man Triphenylphosphin, Tritolylphosphin, Bis(diphenylphosphino)ethan, 1,3-Bis(diphenylphosphino)propan oder 1,4-Bis(diphenylphosphino)butan, insbesondere Triphenylphosphin einsetzen.

Nach einer besonderen Ausführungsform enthält der Palladiumkatalysator eine Bis(triphenylphosphin)-palladium(II)-Verbindung, beispielsweise Bis(triphenylphosphin)-palladium(II)-chlorid oder Bis(triphenylphosphin)-palladium(II)-bromid.

Man setzt den Palladiumkatalysator üblicherweise entsprechend einer Menge von 0,00001 bis 0,3 Mol Palladium, insbesondere 0,000025 bis 0,2 Mol Palladium, bevorzugt 0,00005 bis 0,1 Mol Palladium je Mol 1,2-Bis-halogenmethylbenzol ein.

In einer Vielzahl von Fällen genügt es, die Umsetzung bei einem CO-Druck von 0,5 bis 20 MPa, insbesondere 0,8 bis 10, bevorzugt 1,0 bis 6 MPa durchzuführen.

Üblicherweise kann man die Umsetzung bei einer Temperatur von 50 bis 170°C, insbesondere 70 bis 160°C, bevorzugt 90 bis 150°C mit gutem Erfolg durchführen.

Als dipolar aprotisches Lösungsmittel eignet sich Dioxan, Tetrahydrofuran, ein N-(C₁-C₁₈Alkyl)pyrrolidon, Ethylenglykoldimethylether, ein C₁-C₄-Alkylester einer aliphatischen C₁-C₆-Carbonsäure, ein C₁-C₆-Dialkylether, ein N,N-Di-(C₁-C₄alkyl)amid einer aliphatischen C₁-C₄-Carbonsäure, Sulfolan, ein 1,3-Di-(C₁-C₈-alkyl)-2-imidazotidinon, ein N-(C₁-C₈-Alkyl)caprolactam, ein N,N,N',N'-Tetra-(C₁-C₈alkyl)hamstoff, ein 1,3-Di-(C₁-C₈-alkyl)-3,4,5,6-tetrahydro-2(1H)-pyrimidon, ein N,N,N',N'-Tetra-(C₁-C₈-alkyl)sulfamid, 4-Formylmorpholin, 1-Formylpiperidin oder 1-Formylpyrrolidin, insbesondere ein N-(C₁-C₁₈-Alkyl)pyrrolidon, ein N,N-Di-(C₁-C₄-Alkyl)amid einer aliphatischen C₁-C₄-Carbonsäure, 4-Formylmorpholin, 1-Formylpiperidin oder 1-Formylpyrrolidin, bevorzugt N-Methylpyrrolidon, N-Octylpyrrolidon, N-Dodecylpyrrolidon, N,N-Dimethylformamid, N,N-Dimethylacetamid, 4-Formylmorpholin, 1-Formylpiperidin oder 1-Formylpyrrolidin, besonders bevorzugt N-Methylpyrrolidon, N,N-Dimethylformamid oder N,N-Dimethylacetamid, ganz besonders bevorzugt N-Methylpyrrolidon. Es lassen sich auch Mischungen der vorstehend genannten dipolar aprotischen Lösungsmittel verwenden.

Man kann in das Verfahren Wasser entsprechend einer Menge von 0,5 bis 50 Mol, je Mol 1,2-Bis-halogenmethylbenzol der Formel (II) einsetzen. Üblicherweise führt man die Umsetzung mit einer Menge Wasser entsprechend 1 bis 10, insbesondere 1 bis 4, bevorzugt 1 bis 3 Mol Wasser je Mol 1,2-Bis-halogenmethylbenzol der Formel (II) durch.

Nach einer besonderen Ausführungsform des erfindungsgemäßen Verfahrens legt man das 1,2-Bis-halogenmethylbenzol der Formel (II), den Palladiumkatalysator, das dipolar aprotische Lösungsmittel und gegebenenfalls das ionische Halogenid vor, stellt den CO-Druck und die Temperatur ein und dosiert anschließend Wasser oder ein aus Wasser und dipolar aprotischem Lösungsmittel bestehendes Gemisch zu.

Während der Umsetzung sorgt man für eine gute Durchmischung der Reaktionspartner, um einen zügigen Reaktionsverlauf zu gewährleisten.

Das erfindungsgemäße Verfahren eignet sich sowohl für eine kontinuierliche als auch diskontinuierliche Durchführung.

Die Reaktion wird in der Regel bei einem H₀-Wert ≤ 7 durchgeführt, insbesondere bei H₀ = -3 bis 7, bevorzugt bei -2 bis 6. Es ist allerdings auch möglich, die Reaktion bei einem H₀-Wert von -1 bis 5, insbesondere bei -1 bis 4 durchzuführen. Der H₀-Wert, der ein Maß für die Acidität eines Lösungsmittel ist und für den für verdünnte Lösungen H₀≈ pH gilt, ist im Hollemann-Wiberg "Lehrbuch der Anorganischen Chemie", 91-100. Auflage, Verlag Walter de Gruyter, Berlin 1985, auf den Seiten 246-248 beschrieben. Kislina et al. beschreiben zum Beispiel die Acidität von HCI in N,N-Dimethylformamid in Russ. Chem. Bull., 1994, Vol. 43, auf den Seiten 960-963 In der Regel stellt sich der entsprechende H₀-Wert im Verlauf einer Reaktion von selbst ein, so daß zusätzliche Maßnahmen zur Einstellung des H₀-Wertes meistens nicht erforderlich sind.

Der H₀-Wert geht aus nachfolgender Gleichung hervor:
H₀ = pK_{S,In} + log C_{In}/C_{InH}⁺ (Hammettsche Aciditätsfunktion). Hierbei steht In für die Indikatorbase und InH⁺ für die protonierte Form der Indikatorbase.

Die nachfolgenden Beispiele beschreiben die Erfindung, ohne sie zu beschränken.

### Experimenteller Teil

### Beispiel 1

### Herstellung von Isochroman-3-on

700 g 1,2-Bis-chlormethylbenzol, 2,8 g Bis-(Triphenylphosphin)-palladium(II)-chlorid und 2,4 g Triphenylphosphin werden in 1000 ml Dimethylformamid gelöst und in einem 3000 ml Autoklaven aus HC 4 Stahl unter Schutzgasatmosphäre (Argon) vermischt. Anschließend wird Kohlenmonoxid mit einem Druck von 3 MPa hinzugegeben und die Temperatur auf 130°C erhöht.

Bei 130°C und 4 MPa Druck werden innerhalb von 2 Stunden 400 ml eines Gemisches aus Wasser/Dimethylformamid (Gewichtsverhältnis 2,5 : 1,5) zudosiert. Der Reaktionsdruck wird während der Zudosierung konstant auf 4 MPa gehalten. Nach dem Zudosieren läßt man eine Stunde unter Rühren nachreagieren, kühlt auf 90°C ab und entleert den Autoklaven.

Man erhält 2065 g Reaktionsmischung. Eine gaschromatographische Analyse zeigt, daß die Reaktionsmischung 408 g lsochroman-3-on enthält, entsprechend einer Ausbeute von 70 % lsochroman-3-on, bezogen auf eingesetztes 1,2-Bis-chlormethylbenzol (TON = 700; TOF = 255 x h ⁻¹).

Aus dem Reaktionsgemisch werden in einem Dünnschichtverdampfer bei 100°C und 10 mbar 733 g Leichtsieder abgetrennt.

Der das lsochroman-3-on enthaltende Rückstand wird mit Wasser versetzt. Dabei fällt lsochroman-3-on mit ca. 90 %iger Reinheit (¹H-NMR-Analyse) aus. Durch Destillation erhält man 281 g lsochroman-3-on mit einem Schmelzpunkt von 79 bis 80°C (Reinheit 97 %).
¹H-NMR (300 MHz CdCl₃): δ = 7,20 bis 7,40 (m, 4H), 5,31 (s, 2H), 3,71 (s, 2H).

### Beispiel 2

### Herstellung von Isochroman-3-on

52,5 g 1,2-Bis-chlormethylbenzol, 10,8 g Wasser, 0,21 g Bis(triphenylphosphin)-palladium(II)-chlorid und 0,18 g Triphenylphosphin werden in 100 ml N-Methylpyrrolidon gelöst und in einem 200 ml Autoklaven aus HC 4 Stahl unter Schutzgasatmosphäre (Argon) vermischt.

Anschließend wird Kohlenmonoxid mit einem Druck von 3 MPa hinzugegeben und die Temperatur auf 130°C erhöht. Der Reaktionsdruck wird während der Reaktionsdauer zwischen 3,5 und 4,0 MPa gehalten. Nach 3 Stunden Reaktionszeit läßt man eine Stunde nachrühren, kühlt auf 90°C ab und entleert den Autoklaven.

Man erhält 150 g Reaktionsmischung. Eine gaschromatographische Analyse zeigt, daß die Reaktionsmischung 20,4 g lsochroman-3-on enthält (Ausbeute 46 %; TON = 460; TOF = 153 h⁻¹).

### Vergleichsbeispiel 1

52,5 g 1,2-Bis-chlormethylbenzol, 1,05 g Bis(triphenylphosphin)-palladium(II)-chlorid und 0,87 g Triphenylphosphin werden in 100 ml tert.-Butanol vermischt und in einem 500 ml Glasautoklaven unter Schutzgasatmosphäre (Argon) mit 48,0 g Wasser und 46,8 g Calciumhydroxid suspendiert.

Anschließend wird unter Kohlenmonoxidatmosphäre die Temperatur auf 70°C erhöht. Die unter starker Rührung bei 60°C angefangene Kohlenmonoxidaufnahme ist nach 2 Stunden beendet und man erhält einen dickflüssigen Brei. Die Zugabe von weiteren 1,05 g Bis(triphenylphosphin)-palladium(II)-chlorid und 0,87 g Triphenylphosphin führt zu keiner weiteren Kohlenmonoxidaufnahme.

Nach dem Abkühlen wird mit Salzsäure auf pH 1 angesäuert und mit Diethylether extrahiert. Man erhält 383 g Wasserphase und 299 g organische Phase. Die Wasserphase enthält nach gaschromatographischer Analyse kein Isochroman-3-on, und die organische Phase enthält nach gaschromatographischer Analyse 10,5 g lsochroman-3-on (Ausbeute = 24 %; TON = 46; TOF = 15,4 x h⁻¹).

### Beispiel 3

35,0 g 1,2-Bis-chlormethylbenzol, 140 mg Bis(triphenylphosphin)-palladium(ll)-chlorid und 120 mg Triphenylphosphin werden in 80 ml N-Methylpyrrolidon gelöst und in einem 200 ml Autoklaven aus HC 4 Stahl unter Schutzgasatmosphäre (Argon) vermischt.

Anschließend wird Kohlenmonoxid mit einem Druck von 3,0 MPa hinzugegeben und die Temperatur auf 130°C erhöht. Bei 130°C und 3,5 MPa bis 4,0 MPa Druck wird innerhalb von 3 Stunden 32,5 ml Wasser/N-Methylpyrrolidon (Gewichtsverhältnis 1:5) zudosiert. Nach dem Zudosieren kühl man auf 75°C ab und entspannt den Autoklaven.

Man erhält 162 g Reaktionsmischung. Eine gaschromatographische Analyse zeigt, daß die Reaktionsmischung 17,2 g lsochroman-3-on enthält (Ausbeute 58 %; TON = 584; TOF = 195 x h⁻¹).

### Vergleichsbeispiel 2

Herstellung von lsochroman-3-on unter Verwendung von Pyridin als Wasserstoffhalogenidaufnahmemittel.

Man arbeitet wie in Beispiel 3 beschrieben, setzt jedoch statt 80 ml des dipolar aprotischen Lösungsmittels N-Methylpyrrolidon 80 ml Pyridin als Wasserstoffhalogenidaufnahmemittel und Lösungsmittel ein.

Man erhält 152 g einer aus zwei Phasen bestehenden Reaktionsmischung. Eine gaschromatographische Analyse zeigt, daß in keiner der beiden Phasen lsochroman-3-on enthalten ist. Trotz der Anwesenheit eines Wasserstoffhalogenidaufnahmemittels hat sich kein lsochroman-3-on gebildet.

### Beispiel 4

20,5 g 1,2-Bis-chlormethylbenzol, 70 mg Bis(triphenylphosphin)-palladium(II)-chlorid und 60 mg Triphenylphosphin werden in 50 ml N,N-Dimethylformamid gelöst und in einem 200 ml Autoklaven aus HC 4 Stahl unter Schutzgasatmosphäre (Argon) vermischt.

Anschließend wird Kohlenmonoxid mit einem Druck von 1,7 MPa hinzugegeben und die Temperatur auf 130°C erhöht. Bei 130°C und 2,3 MPa Druck wird innerhalb von 5 Stunden 15 ml Wasser/Dimethylformamid (Gewichtsverhältnis 1:4) zudosiert. Nach dem Zudosieren läßt man eine Stunde nachrühren und entspannt den Autoklaven. Anschließend werden 15 ml Wasser zugegeben und man läßt unter Rühren auf Raumtemperatur abkühlen.

Man erhält 95,8 g Reaktionsmischung. Eine gaschromatographische Analyse zeigt, daß die Reaktionsmischung 13 g lsochroman-3-on enthält (was einer Ausbeute von 75 % entspricht, TON = 900; TOF = 180 x h⁻¹).

### Beispiel 5

87,2 g 1,2-Bis-chlormethylbenzol, 175 mg Bis(triphenylphosphin)-palladium(II)-chlorid und 150 mg Triphenylphosphin werden in 79,8 g N,N-Dimethylformamid gelöst und in einem 300 ml Autoklaven aus HC 4 Stahl unter Schutzgasatmosphäre (Argon) vermischt.

Anschließend wird Kohlenmonoxid mit einem Druck von 3,0 MPa hinzugegeben und die Temperatur auf 130°C erhöht. Bei 130°C und 3,5 bis 4,0 MPa Druck wird innerhalb von 5,6 Stunden 53 ml Wasser/N,N-Dimethylformamid (Gewichtsverhältnis 1:1) zudosiert. Nach dem Zudosieren kühlt man ab und entspannt den Autoklaven.

Man erhält 222 g Reaktionsmischung. Eine gaschromatographische Analyse nach Zusatz von 15 g Wasser zeigt, daß die Reaktionsmischung 36,1 g Isochroman-3-on enthält (Ausbeute: 49 %; TON = 982; TOF = 175 x h⁻¹).

### Beispiel 6

35,0 g 1,2-Bis-chlormethylbenzol und 35 mg Palladium(II)-chlorid werden in 103 g N-Methylpyrrolidon gelöst und in einem 200 ml Autoklaven aus HC 4 Stahl unter Schutzgasatmosphäre (Argon) vermischt.

Anschließend wird Kohlenmonoxid mit einem Druck von 16,0 MPa hinzugegeben und die Temperatur auf 150°C erhöht. Bei 150°C und 1,8 MPa bis 2,2 MPa Druck wird innerhalb von 230 Minuten 7,2 ml Wasser/N-Methylpyrrolidon (Gewichtsverhältnis 1:1) zudosiert. Anschließend bricht man den Versuch ab, indem man entspannt und innerhalb von 15 Minuten auf 50°C abkühlt.

Man erhält 150 g Reaktionsmischung. Eine gaschromatographische Analyse zeigt, daß die Reaktionsmischung 11,4 g 1,2-Bis-chlormethylbenzol und 14,9 g lsochroman-3-on enthält (Selektivität 75 %; Umsatz 67 %; TON = 503; TOF = 131 x h⁻¹).

### Beispiel 7

35,0 g 1,2-Bis-chlormethylbenzol, 35 mg Palladium(II)-chlorid und 100 mg Octyltrimethylammoniumchlorid werden in 103 g N-Methylpyrrolidon gelöst und in einem 200 ml Autoklaven aus HC 4 Stahl unter Schutzgasatmosphäre (Argon) vermischt.

Anschließend wird Kohlenmonoxid mit einem Druck von 16,0 MPa hinzugegeben und die Temperatur auf 150°C erhöht. Bei 150°C und 1,8 MPa bis 2,2 MPa Druck wird innerhalb von 230 Minuten 7,2 ml Wasser/N-Methylpyrrolidon (Gewichtsverhältnis 1:1) zudosiert. Anschließend bricht man den Versuch ab, indem man entspannt und innerhalb von 15 Minuten auf 50°C abkühlt.

Man erhält 149 g Reaktionsmischung. Eine gaschromatographische Analyse zeigt, daß die Reaktionsmischung 15,1 g 1,2-Bis-chlormethylbenzol und 13,5 g lsochroman-3-on enthält (Selektivität 80 %; Umsatz 57 %; TON = 456; TOF = 119 x h⁻¹).

### Beispiel 8

### Herstellung von Isochroman-3-on

299 g 1,2-Bis-chlormethylbenzol und 75 mg Palladium(II)-chlorid werden in 880 g N-Methylpyrrolidon gelöst und in einem 2000 ml Autoklaven aus HC 4 Stahl unter Schutzgasatmosphäre (Argon) vermischt. Anschließend wird Kohlenmonoxid mit einem Druck von 1,5 MPa hinzugegeben und die Temperatur auf 150°C erhöht. Bei 150°C und 2 MPa Druck werden innerhalb von 140 Minuten 123 g eines Gemisches aus Wasser/N-Methylpyrrolidon (Gewichtsverhältnis 1:1) zudosiert. Der Reaktionsdruck wird während der Zudosierung konstant auf 2 MPa gehalten. Nach dem Zudosieren läßt man 20 Minuten unter Rühren nachreagieren, läßt langsam den Druck ab und spült mit Stickstoff. Anschließend wird der Autoklav auf Raumtemperatur abgekühlt und entleert.

Man erhält 1301 g Reaktionsmischung. Eine HPLC-Analyse zeigt, daß die Reaktionsmischung 164 g lsochroman-3-on enthält, entsprechend einer Ausbeute von 65 % lsochroman-3-on, bezogen auf eingesetztes 1,2-Bis-chlormethylbenzol (TON = 4466; TOF = 1675 x h⁻¹).

### Beispiel 9

### Kontinuierliche Herstellung von lsochroman-3-on

299 g 1,2-Bis-chlormethylbenzol und 75 mg Palladium(II)-chlorid werden in 880 g N-Methylpyrrolidon gelöst und in einem 2000 ml Autoklaven aus HC 4 Stahl unter Schutzgasatmosphäre (Argon) vermischt. Anschließend wird Kohlenmonoxid mit einem Druck von 1,5 MPa hinzugegeben und die Temperatur auf 150°C erhöht. Bei 150°C und 2 MPa Druck werden innerhalb von 60 Minuten 45,5 g eines Gemisches aus Wasser/N-Methylpyrrolidon (Gewichtsverhältnis 1:1) zudosiert. In den folgenden 30 Minuten werden weitere 4 g des Gemisches aus Wasser/N-Methylpyrrolidon (Gewichtsverhältnis 1:1) zudosiert. Die gaschromatographische Analyse einer Probe zeigt, daß das Reaktionsgemisch 7,5 Gew.-% 3-lsochromanon, und 13,3 Gew.-% 1,2-Bis-chlormethylbenzol enthält.
Anschließend wird ein Gemisch bestehend aus 24,1 Gew.-% 1,2-Bis-chlormethylbenzol, 0,0060 Gew.-% Palladium(II)-chlorid und 2,46Gew.-% Wasser in N-Methylpyrrolidon kontinuierlich mit einer Rate von 360 g x h⁻¹ zudosiert und gleichzeitig 360 g x h⁻¹ des Reaktionsgemisches aus dem Autoklav abgelassen. Die gaschromatographische Analyse einer Probe der Reaktionsmischung nach einer weiteren Stunde - nachdem 360 g des Reaktionsgemisches abgenommen sind-, zeigt, daß diese 7,2 Gew.-% 3-lsochromanon und 13,4 Gew.-% 1,2-Bis-chlormethylbenzol enthält (für die erste Stunde der kontinuierlichen Fahrweise: TON = 1410; TOF = 1410 x h⁻¹). Anschließend wird die kontinuierliche Zudosierung und Abnahme abgebrochen, der Autoklav entspannt und auf Raumtemperatur abgekühlt. Der Reaktionsdruck wird während der gesamten Reaktionszeit konstant auf 2 MPa gehalten.

## Patentansprüche

1. Verfahren zur Herstellung eines lsochroman-3-ons der Formel (I) 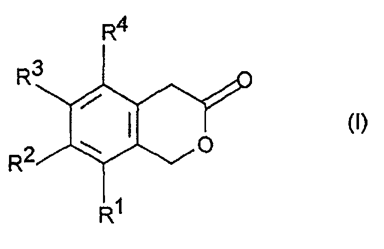 durch Umsetzung eines 1,2-Bis-halogenmethylbenzols der Formel (II) 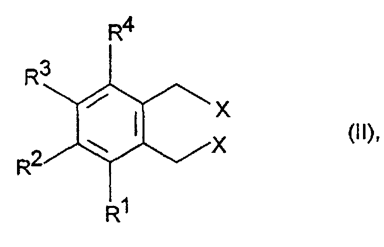 worin X Chlor, Brom oder Jod ist, mit Kohlenmonoxid und Wasser bei einem CO-Druck von 0,1 bis 50 MPa und einer Temperatur von 20 bis 200°C in Anwesenheit oder Abwesenheit eines ionischen Halogenids, in Gegenwart eines Palladiumkatalysators und eines dipolar aprotischen Lösungsmittels, wobei in den Formeln (I) und (II) die Reste R¹, R², R³ und R⁴ unabhängig voneinander darstellen:
ein Wasserstoff- oder Fluoratom;
eine HO₂CCH=CH-, NC- oder F₃C-Gruppe;
einen Alkyl-, Alkoxy- oder Acyloxyrest, mit jeweils 1 bis 18 Kohlenstoffatomen; oder
einen C₆-C₁₈-Aryloxy-, Aryl- oder Heteroarylrest, wobei als Heteroatome 1 bis 3 Atome aus der Gruppe O, N und/oder S vorhanden sind;
einen R⁵₂P(=O)-, R⁶C(=O)-, R⁶OC(=O)-, R⁶OC(=O)CH=CH-, R⁷C(=O)-, R⁷OC(=O)CH=CH- oder R⁷₂P(=O)-Rest; worin R⁵ einen C₁-C₄-Alkylrest, R⁶ einen C₁-C₁₈-Alkylrest und R⁷ einen C₆-C₁₈-Arylrest darstellen; oder worin mindestens einer der Reste R¹, R², R³ und R⁴ durch einen Rest R⁸ substituiert ist, wobei R⁸ die gleiche Bedeutung hat wie R¹; oder worin mindestens zwei der Reste R¹, R², R³ und R⁴ untereinander verknüpft sind und mindestens einen aliphatischen oder aromatischen Ring mit 5 bis 18 C-Atomen bilden.

2. Verfahren nach Anspruch 1, **dadurch gekennzeichnet, daß** die Reste R¹, R², R³ und R⁴ unabhängig voneinander Wasserstoff, Fluor, C₁-C₄-Alkyl oder C₁-C₄-Alkoxy bedeuten oder zwei der Reste R¹, R², R³ und R⁴ untereinander verknüpft sind und einen aliphatischen oder aromatischen Ring mit 5 bis 10 C-Atomen bilden.

3. Verfahren nach Anspruch 1 oder 2, **dadurch gekennzeichnet, daß** zwei, drei oder vier der Reste R¹, R², R³ und R⁴ Wasserstoff bedeuten.

4. Verfahren nach einem oder mehreren der Ansprüche 1 bis 3, **dadurch gekennzeichnet, daß** das ionische Halogenid ein Alkali-, Ammonium- oder Phosphoniumhalogenid ist, wobei Halogenid die Bedeutung Chlorid, Bromid oder lodid hat.

5. Verfahren nach einem oder mehreren der Ansprüche 1 bis 4, **dadurch gekennzeichnet, daß** der Palladiumkatalysator mindestens eine Palladium-(II)-Verbindung, insbesondere PdCl₂, PdBr₂ oder Pd(OAc)₂, oder mindestens eine Palladium-(O)-Verbindung, insbesondere Pd₂dba₃, Pd(P(C₆H₅)₃)₄ oder Pd(η⁴-C₈H₁₂)₂ enthält.

6. Verfahren nach Anspruch 5, **dadurch gekennzeichnet, daß** der Palladiumkatalysator zusätzlich einen Liganden enthält, insbesondere eine Phosphinverbindung.

7. Verfahren nach Anspruch 6, **dadurch gekennzeichnet, daß** die Phosphinverbindung Triphenylphosphin, Tritolylphosphin, Bis(diphenylphosphino)ethan, 1,3-Bis(diphenylphosphino)propan oder 1,4-Bis(diphenylphosphino)butan ist.

8. Verfahren nach einem oder mehreren der Ansprüche 1 bis 7, **dadurch gekennzeichnet, daß** der Palladiumkatalysator Bis(triphenylphosphin)-palladium(II)-chlorid oder Bis(triphenylphosphin)palladium(II)-bromid enthält.

9. Verfahren nach einem oder mehreren der Ansprüche 1 bis 8, **dadurch gekennzeichnet, daß** das dipolar aprotische Lösungsmittel Dioxan, Tetrahydrofuran, ein N-(C₁-C₁₈-Alkyl)pyrrolidon, Ethylenglykoldimethylether, ein C₁-C₄-Alkylester einer aliphatischen C₁-C₆-Carbonsäure, ein C₁-C₆-Dialkylether, ein N,N-Di-(C₁-C₄alkyl)amid einer aliphatischen C₁-C₄-Carbonsäure, Sulfolan, 1,3-Di-(C₁-C₈-alkyl)-2-imidazolidinon, ein N-(C₁-C₈-Alkyl)caprolactam, ein N,N,N',N'-Tetra-(C₁-C₈alkyl)harnstoff, ein 1,3-Di-(C₁-C₈-alkyl)-3,4,5,6-tetrahydro-2(1H)-pyrimidon, ein N,N,N',N'-Tetra-(C₁-C₈-alkyl)sulfamid, 4-Formylmorpholin, 1-Formylpiperidin oder 1-Formylpyrrolidin ist.

10. Verfahren nach einem oder mehreren der Ansprüche 1 bis 9, **dadurch gekennzeichnet, daß** das dipolar aprotische Lösungsmittel N-Methylpyrrolidon, N-Octylpyrrolidon, N-Dodecylpyrrolidon, N,N-Dimethylformamid, N,N-Dimethylacetamid, 4-Formylmorpholin, 1-Formylpiperidin oder 1-Formylpyrrolidin ist.

11. Verfahren nach einem der Ansprüche 1 bis 10, **dadurch gekennzeichnet, daß** man Wasser entsprechend einer Menge von 1 bis 10 Mol, je Mol 1,2-Bis-halogenmethylbenzol der Formel (II), einsetzt.

## Claims

1. A process for preparing a isochroman-3-one of the formula (I) 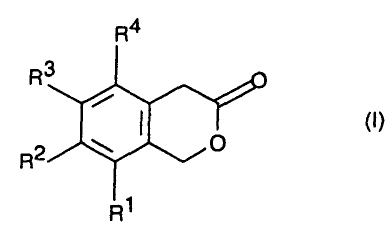 by reacting a 1,2-bishalomethylbenzene of the formula (II) 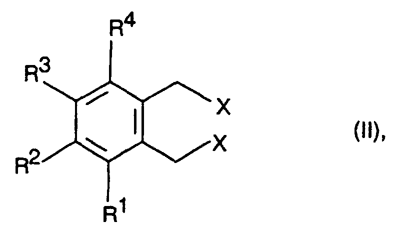 in which X is chlorine, bromine or iodine with carbon monoxide and water at a CO pressure of from 0.1 to 50 MPa and a temperature of from 20 to 200°C in the presence or absence of an ionic halide, in the presence of a palladium catalyst and a dipolar aprotic solvent, where in the formulae (I) and (II) the radicals R¹, R², R³ and R⁴ independently of one another denote:
a hydrogen or fluorine atom;
a HO₂CCH=CH-, NC- or F₃C group;
an alkyl, alkoxy or acyloxy radical having in each case 1 to 18 carbon atoms; or a C₆-C₁₈-aryloxy, aryl or heteroaryl radical, where the heteroatoms present are 1 to 3 atoms from the group O, N and/or S;
a R⁵₂P(=O)-, R⁶C(=O)-, R⁶OC(=O)-, R⁶OC(=O)CH=CH-, R⁷C(=O)-, R⁷OC(=O)CH=CH- or R⁷₂P(=O) radical; in which R⁵ is a C₁-C₄-alkyl radical, R⁶ is a C₁-C₁₈-alkyl radical and R⁷ is a C₆-C₁₈-aryl radical; or in which at least one of the radicals R¹, R², R³ and R⁴ is substituted by a radical R⁸, where R⁸ has the same meaning as R¹; or where at least two of the radicals R¹, R², R³ and R⁴ are linked with one another and form at least one aliphatic or aromatic ring having 5 to 18 carbon atoms.

2. The process as claimed in claim 1, wherein the radicals R¹, R², R³ and R⁴ independently of one another are hydrogen, fluorine, C₁-C₄-alkyl or C₁-C₄-alkoxy, or two of the radicals R¹, R², R³ and R⁴ are linked with one another and form an aliphatic or aromatic ring having 5 to 10 carbon atoms.

3. The process as claimed in claim 1 or 2, wherein two, three or four of the radicals R¹, R², R³ and R⁴ are hydrogen.

4. The process as claimed in one or more of claims 1 to 3, wherein the ionic halide is an alkali metal halide, ammonium halide or phosphonium halide, where halide is chloride, bromide or iodide.

5. The process as claimed in one or more of claims 1 to 4, wherein the palladium catalyst comprises at least one palladium(II) compound, in particular PdCl₂, PdBr₂ or Pd(OAc)₂, or at least one palladium(O) compound, in particular Pd₂dba₃, Pd(P(C₆H₅)₃)₄ or Pd(η⁴-C₈H₁₂)₂.

6. The process as claimed in claim 5, wherein the palladium catalyst additionally comprises a ligand, in particular a phosphine compound.

7. The process as claimed in claim 6, wherein the phosphine compound is triphenylphosphine, tritolylphosphine, bis(diphenylphosphino)ethane, 1,3-bis(diphenylphosphino)propane or 1,4-bis(diphenylphosphino)butane.

8. The process as claimed in one or more of claims 1 to 7, wherein the palladium catalyst comprises bis(triphenylphosphine)palladium(II) chloride or bis(triphenylphosphine)palladium(II) bromide.

9. The process as claimed in one or more of claims 1 to 8, wherein the dipolar aprotic solvent is dioxane, tetrahydrofuran, an N-(C₁-C₁₈-alkyl)pyrrolidone, ethylene glycol dimethyl ether, a C₁-C₄-alkyl ester of an aliphatic C₁-C₆-carboxylic acid, a C₁-C₆-dialkyl ether, an N,N-di-(C₁-C₄-alkyl)amide of an aliphatic C₁-C₄-carboxylic acid, sulfolane, 1,3-di-(C₁-C₈-alkyl)-2-imidazolidinone, an N-(C₁-C₈-alkyl)caprolactam, an N,N,N',N'-tetra-(C₁-C₈-alkyl)urea, a 1,3-di-(C₁-C₈-alkyl)-3,4,5,6-tetrahydro-2(1H)-pyrimidone, an N,N,N',N'-tetra-(C₁-C₈-alkyl)sulfamide, 4-formylmorpholine, 1-formylpiperidine or 1-formylpyrrolidine.

10. A process as claimed in one or more of claims 1 to 9, wherein the dipolar aprotic solvent is N-methylpyrrolidone, N-octylpyrrolidone, N-dodecylpyrrolidone, N,N-dimethylformamide, N,N-dimethylacetamide, 4-formylmorpholine, 1-formylpiperidine or 1-formylpyrrolidine.

11. The process as claimed in one or more of claims 1 to 10, wherein water is employed in an amount corresponding to from 1 to 10 mol per mol of 1,2-bishalomethylbenzene of the formula (II).

## Revendications

1. Procédé pour la préparation d'une isochromane-3-one de formule (I) 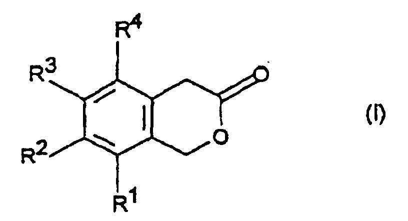 par réaction d'un 1,2-bis-halogénométhylbenzène de formule (II) 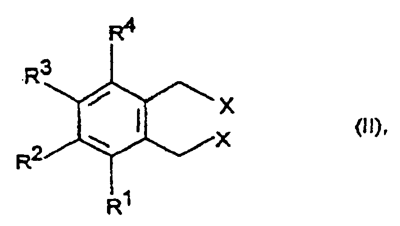 dans laquelle X est le chlore, le brome ou l'iode, avec du monoxyde de carbone et de l'eau à une pression de CO de 0,1 à 50 MPa et une température de 20 à 200°C en présence ou en l'absence d'un halogénure ionique, en présence d'un catalyseur au palladium et d'un solvant dipolaire aprotique, dans les formules (I) et (II) les groupes R¹, R², R³ et R⁴ représentant indépendamment les uns des autres :
un atome d'hydrogène ou un atome de fluor ;
un groupe HO₂CCH=CH-, NC- ou F₃C- ;
un groupe alkyle, alcoxy ou acyloxy, avec respectivement 1 à 18 atomes de carbone, ou un groupe aryloxy, aryle ou hétéroaryle en C₆-C₁₈, dans lesquels sont présents comme hétéroatomes 1 à 3 atomes choisis parmi O, N et/ou S ;
un groupe R⁵₂P(=O)-, R⁶C(=O)-, R⁶OC(=O)-, R⁶OC(=O)CH=CH-, R⁷C(=O)-, R⁷OC(=O)CH=CH- ou R⁷₂P(=O)- ; dans lesquels R⁵ représente un groupe alkyle en C₁-C₄, R⁶ représente un groupe alkyle en C₁ à C₁₈ et R⁷ un groupe aryle en C₆-C₁₈ ;ou dans lesquels au moins un des groupes R¹, R², R³ et R⁴ est substitué par un groupe R⁸, dans lequel R⁸ a la même signification que R¹ ; ou dans lesquels au moins deux des groupes R¹, R², R³ et R⁴ sont couplés entre eux et forment au moins un cycle aliphatique ou aromatique avec 5 à 18 atomes de carbone.

2. Procédé selon la revendication 1, **caractérisé en ce que** les groupes R¹, R², R³ et R⁴ représentent indépendamment les uns des autres un atome d'hydrogène, de fluor, un groupe alkyle en C₁ à C₄ ou alcoxy en C₁ à C₄ ou deux des groupes R¹, R², R³ et R⁴ sont couplés ensemble et forment un cycle aliphatique ou aromatique ayant de 5 à 10 atomes de carbone.

3. Procédé selon la revendication 1 ou 2, **caractérisé en ce que** deux, trois ou quatre des groupes R¹, R², R³ et R⁴ représentent un atome d'hydrogène.

4. Procédé selon une ou plusieurs des revendications 1 à 3, **caractérisé en ce que** l'halogénure ionique est un halogénure de métal alcalin, d'ammonium ou de phosphonium, dans lequel halogénure a la signification de chlorure, bromure ou iodure.

5. Procédé selon une ou plusieurs des revendications 1 à 4, **caractérisé en ce que** le catalyseur au palladium contient au moins un composé de palladium-(II), en particulier PdCl₂, PdBr₂ ou Pd(OAc)₂ ou au moins un composé du palladium-(0), en particulier Pd₂dba₃, Pd(P(C₆H₅)₃)₄ ou Pd(η⁴-C₈H₁₂)₂.

6. Procédé selon la revendication 5, **caractérisé en ce que** le catalyseur au palladium contient en plus un ligand, en particulier un composé de phosphine.

7. Procédé selon la revendication 6, **caractérisé en ce que** le composé de phosphine est la triphénylphosphine, la tritolylphosphine, le bis(diphénylphosphino)éthane, le 1,3-bis(diphénylphosphino)propane ou le 1,4-bis(diphénylphosphino)butane.

8. Procédé selon une ou plusieurs des revendications 1 à 7, **caractérisé en ce que** le catalyseur de palladium contient le chlorure de bis(triphénylphosphine)-palladium(II) ou le bromure de bis(triphénylphospine)-palladium(II).

9. Procédé selon une ou plusieurs des revendications 1 à 8, **caractérisé en ce que** le solvant aprotique dipolaire est le dioxane, le tétrahydrofurane, une N-(alkyle en C₁-C₁₈)pyrrolidone, l'éther diméthylique d'éthylèneglycol, un ester d'alkyle en C₁-C₄ d'un acide carboxylique aliphatique en C₁-C₆, un dialkyléther en C₁-C₆, un N,N-dialkyle en C₁-C₄)amide d'un acide carboxylique aliphatique en C₁-C₄, le sulfolane, une 1,3-di-(alkyle en C₁-C₈)-2-imidazolidinone, un N-(alkyle en C₁-C₈)caprolactame, une N,N,N',N'-tétra-(alkyle en C₁-C₈)urée, une 1,3-di-(alkyle en C₁-C₈)-3,4,5,6-tétrahydro-2(1H)-pyrimidone, un N,N,N',N'-tétra-(alkyle en C₁-C₈)sulfamide, la 4-formylmorpholine, la 1-formylpipéridine ou la 1-formylpyrrolidine.

10. Procédé selon une ou plusieurs des revendications 1 à 9, **caractérisé en ce que** le solvant aprotique dipolaire est la N-méthylpyrrolidone, la N-octylpyrrolidone, la N-dodécylpyrrolidone, le N,N-diméthylformamide, le N,N-diméthylacétamide, la 4-formylmorpholine, la 1-formylpipéridine ou la 1-formylpyrrolidine.

11. Procédé selon une des revendications 1 à 10, **caractérisé en ce qu'**on utilise de l'eau correspondant à une quantité de 1 à 10 mol, respectivement par mol de 1,2-bis-halogénométhylbenzène de formule (II).
